# EUROPEAN PATENT APPLICATION

(11) **EP 4 563 657 A1**
(43) Date of publication of application: **04.06.2025**
(21) Application number: 22952244.6
(22) Date of filing: 26.07.2022
(51) Int. Cl.: C08L 75/06, C08L 75/02, A61F 6/04, A41D 19/015, B65B 63/04

(54) **POLYURETHANE-POLYUREA AQUEOUS DISPERSION, AND PREPARATION METHOD THEREFOR AND USE THEREOF**

(71) Applicant: Wanhua Chemical Group Co., Ltd., Yantai, Shandong 264006 (CN)
(72) Inventor: WANG, Zhen, Yantai, Shandong 264000 (CN); JIN, Yunquan, Yantai, Shandong 264000 (CN); JI, Xueshun, Yantai, Shandong 264000 (CN); CAO, Yuyang, Yantai, Shandong 264000 (CN); ZHANG, Xu, Yantai, Shandong 264000 (CN); LI, Weifei, Yantai, Shandong 264000 (CN); WANG, Haimei, Yantai, Shandong 264000 (CN); DENG, Junying, Yantai, Shandong 264000 (CN); SUN, Jiakuan, Yantai, Shandong 264000 (CN)
(74) Representative: Pfenning, Meinig & Partner mbB
(86) International application number: PCT/CN2022/107894
(87) International publication number: WO 2024/020779

(57) **Abstract**

Provided in the present invention are a polyurethane-polyurea aqueous dispersion, and a preparation method therefor and the use thereof. The polyurethane-polyurea aqueous dispersion comprises a unit derived from a component a, wherein the component a comprises at least one polyhydric alcohol with a functionality of 2 or more, and the component a comprises at least 40 wt% or more of a semi-crystalline polyester polyol obtained by reacting a dihydric alcohol with a component I, the component I comprising an aliphatic dicarboxylic acid and a component II at a molar ratio of (2.1-10): 1, and the component II being an aromatic dicarboxylic acid and/or aromatic dicarboxylic acid anhydride. A soft and elastic product prepared on the basis of the polyurethane-polyurea aqueous dispersion provided in the present invention has the comprehensive properties of good elasticity, strength, water resistance, wearability, tactility, etc.

## Description

### TECHNICAL FIELD

The present application relates to the technical field of preparing soft elastic products (e.g., gloves, condoms, etc.), and in particular relates to a polyurethane-polyurea aqueous dispersion which facilitates the preparation of high-performance soft elastic products, a preparation method therefor and use thereof.

### BACKGROUND

Soft elastic products such as disposable gloves are widely used in the fields of medical gloves, examination gloves, household and industry, etc. At present, the materials used in the market for the preparation of soft elastic products (such as disposable gloves, condoms, etc.) mainly include natural latex, chloroprene rubber, nitrile rubber and PVC. Natural latex is prone to allergies in some people because it contains protein, while chloroprene rubber and nitrile rubber products have problems of high odor and production pollution. In comparison, polyurethane dispersion is better than the resin of the above materials in mechanical strength, extensibility, breathability, and glove preparation process of environmental protection. At present, there have been some research reports on the use of polyurethane to produce soft elastic products.

Patent document CN104725590B discloses a low modulus polyurethane dispersion for disposable gloves, which uses polytetrahydrofuran diol, polypropylene glycol, and toluene diisocyanate as the raw materials, and the prepared gloves have performance similar to those of rubber products. However, the product prepared using polypropylene glycol has the problem of poor water resistance, and if exposed to water during use, the strength of which will decrease very significantly, and does not have practical use value.

Patent document CN108864394A discloses a kind of glove prepared by compounding sulfonated polyester and polypropylene glycol for use in the medical fields, claiming that it has excellent alcohol resistance, but in fact, the systems using polypropylene glycol are all unable to realize good alcohol resistance, and the overall strength of the glove is low, so the applicability is not strong.

In patent document CN106188477B, a polyurethane aqueous dispersion with high elasticity was synthesized by using polyether and a high molecular weight resilience agent, and was used in the preparation of gloves, but the high cross-linking structure in the polyurethane dispersion was unfavorable to the elasticity, and the document only evaluated the mechanical performance of the resin, and there was no elaboration of the preparation and performance of the gloves.

### SUMMARY

The present application provides a polyurethane-polyurea aqueous dispersion which is appropriate for preparing soft elastic products. The soft elastic products prepared from the polyurethane-polyurea aqueous dispersion have excellent overall performance including good elasticity, strength, water resistance, and wearing tactility.

To achieve the object, the present application provides the technical solutions below.

The present application provides a polyurethane-polyurea aqueous dispersion, which includes a unit derived from a component a; the component a includes at least one polyol having a functionality of 2 or more, and the component a at least includes 40wt% or more of a semi-crystalline polyester polyol obtained by reacting a diol with a component I, wherein the component I includes an aliphatic dicarboxylic acid and a component II by a molar ratio of 2.1-10: 1, and the component II is an aromatic dicarboxylic acid and/or an aromatic dicarboxylic anhydride.

In some embodiments, the diol used to prepare the semi-crystalline polyester polyol at least includes one or more of 1,4-butanediol and 1,6-hexanediol, and optionally includes an additional diol having a relative molecular mass of less than 150; the additional diol includes one or more of ethylene glycol, 1,3-butanediol, neopentyl glycol, 1,5-pentanediol, propylene glycol, diethylene glycol, and dipropylene glycol; the additional diol accounts for less than 50wt% of the diol used to prepare the semi-crystalline polyester polyol;
and/or, the component II used to prepare the semi-crystalline polyester polyol is selected from one or more of benzenedicarboxylic acid and phthalic anhydride; preferably, the benzenedicarboxylic acid is selected from isophthalic acid and/or phthalic acid; more preferably, the component II is isophthalic acid;
and/or, the aliphatic dicarboxylic acid used to prepare the semi-crystalline polyester polyol is hexanedioic acid.

In some embodiments, the semi-crystalline polyester polyol is an opaque waxy solid at room temperature.

In some embodiments, the polyurethane-polyurea aqueous dispersion is prepared by reacting a raw material including the following components:
component a: including at least one polyol having a functionality of 2 or more; and the semi-crystalline polyester polyol accounts for 40wt% or more of the component a, and preferably the semi-crystalline polyester polyol accounts for 70-100wt% of the component a;
component b: a polyisocyanate;
component c: a hydrophilic compound; the hydrophilic compound contains 2-3 groups having reactivity with NCO, and a hydrophilic group of the hydrophilic compound includes one or more of an ionic group and a latent ionic group;
optional component d: an alcohol chain extender different from the component a; and
component f: a polyamine having a number average molecular mass of 500 g/mol or less.

In some embodiments, in a case where the hydrophilic group of the component c does not include the ionic group, the raw material further includes component e, and the component e is a compound capable of ionizing the component c;
preferably, in a case where the raw material includes the component e, relative to the total weight of the components a, b, c, d, e, and f, proportions of each component used to prepare the polyurethane-polyurea aqueous dispersion are as follows: 56-85wt% of the component a, 10-30wt% of the component b, 0.8-4wt% of the component c, 0-3wt% of the component d, 0.5-4wt% of the component f, and 0.6-4wt% of the component e;
preferably, in a case where the raw material does not include the component e, relative to the total weight of the components a, b, c, d, and f, proportions of each component used to prepare the polyurethane-polyurea aqueous dispersion are as follows: 56-85wt% of the component a, 10-30wt% of the component b, 0.8-4wt% of the component c, 0-3wt% of the component d, and 0.5-4wt% of the component f.

In some embodiments, in the component a, the polyol has a number average molecular mass of 500-15000 g/mol, preferably the polyol is a polyol having a number average molecular mass of 800-10000 g/mol and a functionality of 2 to 4, and more preferably the polyol is a polyol having a number average molecular mass of 1000-5000 g/mol and a functionality of 2 to 3;
and/or, in the component b, the polyisocyanate is selected from one or more of an aromatic polyisocyanate, an aliphatic polyisocyanate, and an alicyclic polyisocyanate, and preferably the polyisocyanate has at least two isocyanate groups;
and/or, in the component c, the groups having reactivity with NCO contained in the hydrophilic compound are hydroxyl and/or amino;
and/or, in the component c, the ionic group is preferably a carboxylate ion and/or a sulfonate ion, and the latent ionic group is preferably carboxyl;
and/or, in the component d, the alcohol chain extender different from the component a is a small-molecule alcohol compound having a relative molecular mass of less than 150;
and/or, the component e is triethylamine;
and/or, the component f is a polyamine having a relative molecular mass of 60-500, and preferably one or more of ethylenediamine, 1,2-diaminopropane, 1,4-diaminobutane, 1,6-hexanediamine, 2-methylpentane-1,5-diamine, isophorone diamine, 4,4-diaminodicyclohexylmethane, piperazine, and diethylenetriamine.

In some embodiments, the hydrophilic compound used as the component c is selected from one or more of dimethylolpropionic acid, dimethylolbutanoic acid, dihydroxysuccinic acid, N-(2-aminoethyl)-2-aminoethanesulfonic acid, N-(3-aminopropyl)-2-aminoethanesulfonic acid, and salts thereof, the salts include one or more of their respective alkaline metal salts, alkaline earth metal salts or ammonium salts, and preferably the component c is dimethylolpropionic acid;
and/or, the alcohol chain extender used as the component d is selected from one or more of ethylene glycol, 1,2-propanediol, 1,3-propanediol, 1,4-butanediol, 1,3-butanediol, 2,3-butanediol, 1,5-pentanediol, 1,6-hexanediol, neopentyl glycol, 1,4-dihydroxycyclohexane, 1,4-dimethylolcyclohexane, 1,8-octanediol, 1,10-decanediol, 1,12-dodecanediol, neopentyl glycol, 1,4-cyclohexanediol, 1,4-cyclohexanedimethanol, and 2-ethyl-1,3-hexanediol; and preferably one or more of 1,2-propanediol, 1,3-propanediol, 1,4-butanediol, 1,3-butanediol, neopentyl glycol, 1,4-cyclohexanedimethanol, and 1,6-hexanediol.

**In** some embodiments, the component a optionally includes an additional polyol different from the semi-crystalline polyester polyol, the additional polyol is selected from one or more of a polyester polyol, a polycaprolactone polyol, a polycarbonate polyol, and a polyether polyol, and the additional polyol accounts for 0-60wt% of the component a.

The present application also provides a preparation method for the polyurethane-polyurea aqueous dispersion described above, wherein the polyurethane-polyurea aqueous dispersion is prepared by reacting a raw material including the component a;
preferably, the raw material further includes component b, component c, optional component d, and component f;
wherein the component b is a polyisocyanate; the component c is a hydrophilic compound, and the hydrophilic compound contains 2-3 groups having reactivity with NCO; the component d is an alcohol chain extender different from the component a; the component f is a polyamine having a number average molecular mass of 500 g/mol or less; in a case where the component c does not include an ionic group, the raw material further includes component e, and the component e is a compound capable of ionizing the component c;
preferably, the preparation method includes a step of obtaining a prepolymer containing isocyanate as terminal group by reacting at 60-90°C.

The present application also provides a soft elastic product; the soft elastic product is produced from the polyurethane-polyurea aqueous dispersion as described above; preferably the soft elastic product includes gloves and/or condoms.

The present application also provides a preparation method for a soft elastic product including a step of subjecting a feeding liquid containing a polyurethane-polyurea aqueous dispersion to a mold required to prepare a soft elastic product; preferably, the soft elastic product includes gloves and/or condoms; the polyurethane-polyurea aqueous dispersion employs the polyurethane-polyurea aqueous dispersion as described above.

The technical solutions provided by the present application have the beneficial effects below.

The polyurethane-polyurea dispersion provided by the present application is obtained based on the polyol component (i.e., component a) at least including 40wt% or more of the semi-crystalline polyester polyol containing a benzene ring structure, and the semi-crystalline polyester polyol is obtained by reacting a diol with an aliphatic dicarboxylic acid and component II (an aromatic dicarboxylic acid and/or an aromatic dicarboxylic anhydride) having a particular molar ratio. The soft elastic products prepared from such polyurethane-polyurea dispersion, such as gloves, condoms, etc., have excellent overall performance, possessing good tensile strength, elongation, resilience, wearing tactility and water resistance at the same time, and has excellent resistance to oxides and polar solvents. The polyurethane-polyurea dispersion based on the present application can be used to prepare soft elastic products suitable for medical, households, and industrial use, such as gloves; it is also particularly suitable for the production of condoms.

### DETAILED DESCRIPTION

In order to facilitate understanding of the present application, the present application will be further described below in terms of embodiments. It should be understood that the following embodiments are only used for a better understanding of the present application and do not imply that the present application is only limited to the following embodiments.

Unless otherwise defined, all technical and scientific terms used herein have the same meaning as commonly understood by those skilled in the art to which the present application belongs. The term "and/or" used herein includes any and all combinations of one or more of the relevant listed items.

Where specific experimental steps or conditions are not indicated in the embodiments, the embodiments may be performed in accordance with the operations or conditions of corresponding conventional experimental steps in the art. The reagents or instruments used without manufacturers indicated are regular products commercially available.

The present application provides, on one aspect, a polyurethane-polyurea aqueous dispersion including a unit derived from a component a; the component a includes at least one polyol having a functionality of 2 or more, and the component a at least includes 40wt% or more of a semi-crystalline polyester polyol obtained by reacting a diol with a component I, wherein the component I includes an aliphatic dicarboxylic acid and a component II by a molar ratio of 2.1-10: 1 (e.g., a molar ratio of 2.1: 1, 2.5: 1, 3: 1, 4.5: 1, 5: 1, 6: 1, 7: 1, 8: 1, 9: 1, 10: 1, etc.), and the component II is an aromatic dicarboxylic acid and/or an aromatic dicarboxylic anhydride.

The inventors have found that when the polyol (component a), at least including 40wt% or more (e.g., 40wt%, 42wt%, 45wt%, 50wt%, 55wt%, 60wt%, 65wt%, 70wt%, 75wt%, 80wt%, 90wt%, 100%, etc.) of a semi-crystalline polyester polyol which is prepared by reacting the diol with an aliphatic dicarboxylic acid and component II (an aromatic dicarboxylic acid and/or an aromatic dicarboxylic anhydride) having a molar ratio of 2.1-10: 1, is used in the preparation of the polyurethane-polyurea aqueous dispersion, the polyurethane-polyurea aqueous dispersion prepared by the scheme of the present application can reduce the crystallinity of polyester and improve elasticity to obtain good strength and other characteristics; the polyurethane-polyurea aqueous dispersion is used in the preparation of soft elastic products, and the polyurethane-polyurea aqueous dispersion obtained is particularly suitable for the preparation of soft elastic products, such as gloves and/or condoms; using the polyurethane-polyurea aqueous dispersion in the preparation of soft elastic products, the resulting soft elastic products have significantly improved overall performances, including excellent tensile strength, elongation, and resilience at the same time, and the material is soft and elastic, bringing an improved wearing tactility. On the contrary, if the semi-crystalline polyester polyol is not used or the proportion of semi-crystalline polyester polyol is less than 40wt% in the polyol (component a), or the semi-crystalline polyester polyol is obtained from an aliphatic dicarboxylic acid and component II (an aromatic dicarboxylic acid and/or an aromatic dicarboxylic anhydride) which do not satisfy the mole ratio of 2.1-10:1, the resulting soft elastic products will have significant deteriorated performance and cannot obtain good overall performance.

In a preferred embodiment, by allowing the semi-crystalline polyester polyol prepared by reacting the diol with the component I to account for 70-100wt% of the component a, soft elastic products with better performance are prone to be obtained.

In some preferred embodiments, the diol used to prepare the semi-crystalline polyester polyol at least includes one or more of 1,4-butanediol and 1,6-hexanediol, and optionally includes an additional diol having a relative molecular mass of less than 150; the additional diol includes one or more of ethylene glycol, 1,3-butanediol, neopentyl glycol, 1,5-pentanediol, propylene glycol, diethylene glycol, and dipropylene glycol; the additional diol accounts for less than 50wt% of the diol used to prepare the semi-crystalline polyester polyol, i.e., its amount is less than 1,4-butanediol and/or 1,6-hexanediol. When preparing the semi-crystalline polyester polyol, the person skilled in the art can easily determine the amount of the diol in the reaction system based on the target molecular mass. In the preparation of semi-crystalline polyester polyol, the diol used is preferably 1,4-butanediol and/or 1,6-hexanediol, or preferably 50wt% (relative to the total weight of the diol) or more of 1,4-butanediol and/or 1,6-hexanediol, which can improve the performance of the resulting soft elastic product.

In some preferred embodiments, the component II used to prepare the semi-crystalline polyester polyol is selected from one or more of benzenedicarboxylic acid and phthalic anhydride; preferably, the benzenedicarboxylic acid is selected from isophthalic acid and/or phthalic acid; more preferably, the component II is isophthalic acid. Isophthalic acid is preferably used which is favorable for obtaining a soft elastic product with superior performance.

In some preferred embodiments, the aliphatic dicarboxylic acid used to prepare the semi-crystalline polyester polyol is hexanedioic acid.

In some preferred embodiments, the diol used to prepare the semi-crystalline polyester polyol at least includes one or more of 1,4-butanediol and 1,6-hexanediol, the component II used is selected from one or more of isophthalic acid, phthalic acid, and phthalic anhydride, and preferably isophthalic acid, and the aliphatic dicarboxylic acid used is hexanedioic acid, and the molar ratio of hexanedioic acid to the component II is 2.1-10:1; the polyurethane-polyurea aqueous dispersion prepared based on the preferred semi-crystalline polyester polyol contributes to better product performance, and the resulting products have significantly improved tensile strength, elongation, resilience, wearing tactility, and water resistance. The semi-crystalline polyester polyol obtained by such preferred method is an opaque waxy solid at room temperature, and the semi-crystalline polyester polyol has a certain degree of regularity, wherein the room temperature refers to 25°C.

The semi-crystalline polyester polyol used in the polyurethane-polyurea aqueous dispersion of the present application can be produced by a conventional preparation process of polyester polyol in the field, or can directly employ a commercially available product meeting the above requirements. Specifically, the semi-crystalline polyester polyol can be prepared by the melting polycondensation method known to those skilled in the art, which can be carried out directly with reference to the existing process, for example, the preparation method for the polyester polyol in patent CN110724249B can be referred to, and specifically refers to the embodiment of the patent; for example, the aliphatic dicarboxylic acid, component II (an aromatic dicarboxylic acid and/or an aromatic dicarboxylic anhydride) and diol are added to a reaction kettle by formula amounts required for the preparation of semi-crystalline polyester polyol, the system is heated up to 130-180°C until the system is transparent, then the system is heated up to 200-230°C for reacting (e.g., the reaction is carried out for about 2-3 h) until the water output is close to the theoretical water output, then a catalyst is added and the temperature is increased to 235-250°C and the vacuum device is turned on, (e.g., under the vacuum pressure of -0.095 Mpa) the reaction is continually carried out until the acid value of the system reaches 0.3 mgKOH/g or lower, then the system is cooled down (e.g., cooled down to about 120°C) to discharge the product, and packaged to obtain the target semi-crystalline polyester polyol; during the reaction process, a catalyst is added to the reaction kettle according to needs; for example, the catalyst is selected from one or more of organotin, organobismuth, organotitanium (such as tetrabutyl titanate, tetraisopropyl titanate, etc.), and the catalyst dosage can be 50-300 ppm of the total mass of the reactants; during the preparation process, the ratio of diacid to diol can be determined according to the molecular mass of the target polyester polyol, and usually, the added amount of diol is 105-120% of the theoretical feeding amount.

**In** some embodiments, the component a optionally includes an additional polyol different from the semi-crystalline polyester polyol. **In** some embodiments, in the component a, the polyol (i.e., the semi-crystalline polyester polyol and the optional additional polyol) has a number average molecular mass of 500-15000 g/mol, preferably the polyol is a polyol having a number average molecular mass of 800-10000 g/mol and a functionality of 2 to 4, and more preferably the polyol is a polyol having a number average molecular mass of 1000-5000 g/mol and a functionality of 2 to 3.

In the component a, the additional polyol involved is optionally selected from one or more of a polyester polyol, a polycaprolactone polyol, a polycarbonate polyol, and a polyether polyol, and the additional polyol accounts for 0-60wt% of the component a. There is no particular limitation on the specific selection of the aforementioned additional polyol, and those polyols conventionally allowed to be added in the polyurethane-polyurea aqueous dispersion may be used.

Specifically, the polyester polyol of the additional polyol can be obtained by, for example, reacting the carboxylic acid and/or anhydride with the polyol through a known preparation process; for example, the polyester polyol can be obtained by subjecting an aliphatic, alicyclic, aromatic dicarboxylic acid, or a polycarboxylic acid or its respective anhydride and a polyol to dehydration condensation. Examples of the carboxylic acid or anhydride include, but are not limited to, succinic acid, methylsuccinic acid, glutaric acid, hexanedioic acid, heptanedioic acid, octanedioic acid, azelaic acid, sebacic acid, nonanedicarboxylic acid, decanedicarboxylic acid, terephthalic acid, isophthalic acid, phthalic acid, tetrahydrophthalic acid, hexahydrophthalic acid, cyclohexanedicarboxylic acid, maleic acid, fumaric acid, malonic acid, trimellitic acid, phthalic anhydride, trimellitic anhydride, succinic anhydride, or mixtures thereof. Examples of the polyol include, but are not limited to, ethylene glycol, 1,2-propanediol, 1,3-propanediol, 1,4-butanediol, 1,3-butanediol, 2,3-butanediol, 1,5-pentanediol, 1,6-hexanediol, 2,2-dimethyl-1,3-propanediol, 1,4-dihydroxycyclohexane, 1,4-dimethylolcyclohexane, 1,8-octanediol, 1,10-decanediol, 1,12-dodecanediol, or mixtures thereof; optionally, a polyol having a higher functionality can be added, such as trimethylolpropane. The polyester polyol may also include a fatty acid-containing polyester having an average OH functionality of about 2, and may also include a product from a transesterification reaction between castor oil and an oil other than castor oil. Preferably, the additional polyol in component a is a polyester polyol having structural components of a diacid containing one or more of isophthalic acid, terephthalic acid, and hexanedioic acid, and a diol containing one or more of neopentyl glycol, ethylene glycol, butylene glycol, and hexanediol.

The polyester polyol of the additional polyol can also be homopolymers or copolymers of lactone, which can be obtained by subjecting a lactone or a lactone mixture and a suitable diol and/or a suitable polyol having a functionality of more than two to a ring opening reaction.

Polycarbonate with hydroxyl groups prepared from the diol and carbonate are also suitable to be the additional polyol in component a. The diol used in the polycarbonate is preferably 1,4-butanediol and/or 1,6-hexanediol, and the carbonate can be a diaryl carbonate and/or a dialkyl carbonate. The diaryl carbonate is, for example, a diphenyl carbonate, and the dialkyl carbonate is, for example, a dimethyl carbonate.

Polyether polyol is also suitable to be the additional polyol in component a. A preferred polyether polyol is polytetrahydrofuran polyol. In a case where the additional polyol is added to the component a, the additional polyol is preferably polytetrahydrofuran polyol, resulting in a better elasticity.

In some embodiments, the polyurethane-polyurea aqueous dispersion is prepared by reacting a raw material including the following components:
component a: including at least one polyol having a functionality of 2 or more; the semi-crystalline polyester polyol accounts for 40wt% or more of the component a,
component b: a polyisocyanate;
component c: a hydrophilic compound; the hydrophilic compound contains 2-3 groups having reactivity with NCO;
optional component d: an alcohol chain extender different from the component a; and
component f: a polyamine having a number average molecular mass of 500 g/mol or less.

**In** a case where the hydrophilic group of the component c does not include the ionic group, the raw material further includes component e, and the component e is a compound capable of ionizing the component c without ionic groups. Component e may not be added in a case where component c contains an ionic group.

**In** some embodiments, in a case where the raw material includes the component e, relative to the total weight of the components a, b, c, d, e, and f, proportions of each component used to prepare the polyurethane-polyurea aqueous dispersion are as follows: 56-85wt% of the component a, 10-30wt% of the component b, 0.8-4wt% of the component c, 0-3wt% of the component d, 0.5-4wt% of the component f, and 0.6-4wt% of the component e;

in a case where the raw material does not include the component e, relative to the total weight of the components a, b, c, d, and f, proportions of each component used to prepare the polyurethane-polyurea aqueous dispersion are as follows: 56-85wt% of the component a, 10-30wt% of the component b, 0.8-4wt% of the component c, 0-3wt% of the component d, and 0.5-4wt% of the component f.

**In** the component b, there is no particular limitation on the specific selection of the polyisocyanate, those polyisocyanates conventionally used in the art may be used. In some embodiments, the polyisocyanate may be selected from one or more of an aromatic polyisocyanate, an aliphatic polyisocyanate, and an alicyclic polyisocyanate, and preferably the polyisocyanate has at least two isocyanate groups. Preferably, the polyisocyanate has a molecular formula of Y(NCO)₂, wherein Y represents a divalent aliphatic hydrocarbon group containing 4 to 12 carbon atoms, a divalent alicyclic hydrocarbon group containing 6 to 15 carbon atoms, a divalent aromatic hydrocarbon group containing 6 to 15 carbon atoms, or a divalent araliphatic hydrocarbon group containing 7 to 15 carbon atoms. In some embodiments, the polyisocyanate may be one or more of tetramethylene diisocyanate, methyl pentamethylene diisocyanate, hexamethylene diisocyanate, dodecamethylene diisocyanate, 1,4-cyclohexane diisocyanate, isophorone diisocyanate, 4,4'-dicyclohexylmethane diisocyanate, 4,4'-dicyclohexylpropane diisocyanate, 1,4-phenylene diisocyanate, 2,4-toluene diisocyanate, 2,6-toluene diisocyanate, 4,4'-diphenylmethane diisocyanate, 2,2'-diphenylmethane diisocyanate, 2,4'-diphenylmethane diisocyanate, tetramethylxylylene diisocyanate, p-xylylene diisocyanate, and p-isopropylidene diisocyanate. The component b may also include a small amount of polyisocyanate having a higher functionality known in polyurethane chemistry, or a modified polyisocyanate containing, for example, a carbodiimide group, an allophanate group, an isocyanurate group, a carbamate group, and/or a biuret group. Preferably, the component b is preferably one or more of hexamethylene diisocyanate, isophorone diisocyanate, dicyclohexylmethane diisocyanate, diphenylmethane diisocyanate, and toluene diisocyanate.

In some embodiments, in the component c, the groups having reactivity with NCO contained in the hydrophilic compound are hydroxyl and/or amino. In some embodiments, in the component c, a hydrophilic group of the hydrophilic compound includes one or more of an ionic group and a latent ionic group, the ionic group is preferably a carboxylate ion and/or a sulfonate ion, and the latent ionic group is preferably carboxyl. In some embodiments, the hydrophilic compound used as the component c is selected from one or more of dimethylolpropionic acid, dimethylolbutanoic acid, dihydroxysuccinic acid, N-(2-aminoethyl)-2-aminoethanesulfonic acid, N-(3-aminopropyl)-2-aminoethanesulfonic acid, and salts thereof, the salts include one or more of their respective alkaline metal salts, alkaline earth metal salts, or ammonium salts, and preferably the component c is dimethylolpropionic acid.

In some embodiments, in the component d, the alcohol chain extender different from the component a is a small-molecule alcohol compound having a relative molecular mass of less than 150. In some embodiments, the alcohol chain extender used as the component d is selected from one or more of ethylene glycol, 1,2-propanediol, 1,3-propanediol, 1,4-butanediol, 1,3-butanediol, 2,3-butanediol, 1,5-pentanediol, 1,6-hexanediol, neopentyl glycol, 1,4-dihydroxycyclohexane, 1,4-dimethylolcyclohexane, 1,8-octanediol, 1,10-decanediol, 1,12-dodecanediol, neopentyl glycol, 1,4-cyclohexanediol, 1,4-cyclohexanedimethanol, and 2-ethyl-1,3-hexanediol, and preferably one or more of 1,2-propanediol, 1,3-propanediol, 1,4-butanediol, 1,3-butanediol, neopentyl glycol, 1,4-cyclohexanedimethanol, and 1,6-hexanediol.

In some embodiments, the component e is a volatile tertiary amine compound, preferably triethylamine.

In some embodiments, the component f is a polyamine having a relative molecular mass of 60-500, and preferably one or more of ethylenediamine, 1,2-diaminopropane, 1,4-diaminobutane, 1,6-hexanediamine, 2-methylpentane-1,5-diamine, isophorone diamine, 4,4-diaminodicyclohexylmethane, piperazine, and diethylenetriamine; preferably one or two of ethylenediamine and isophorone diamine.

In the present application, unless otherwise specified, the meaning of "more than one" or "poly-" refers to more than or equal to two.

The polyurethane-polyurea aqueous dispersion of the present application can be prepared according to the existing preparation process of polyurethane-polyurea aqueous dispersion in the field, and there is no special requirement for this. Specifically, the polyurethane-polyurea aqueous dispersion is prepared by reacting a raw material including the component a; further specifically, the raw material therein further includes component b, component c, optional component d, and component f; wherein the component b is a polyisocyanate; the component c is a hydrophilic compound, and the hydrophilic compound contains 2-3 groups having reactivity with NCO; the component d is an alcohol chain extender different from the component a; the component f is a polyamine having a number average molecular mass of 500 g/mol or less; in a case where the component c does not contain the ionic group, the raw material further includes component e, and the component e is a compound capable of ionizing the component c. Preferably, the preparation method includes a step of obtaining a prepolymer containing isocyanate as terminal group by reacting at 60-90°C. For reference, in a case where the component c does not contain the ionic group, the specific preparation steps include: reacting the component a, the component b, the component c, and the optional component d in the exist of a solvent (e.g., acetone) at 60-90°C to obtain the prepolymer containing isocyanate as terminal group, diluting with a solvent (e.g., acetone) and then adding the component e for neutralization, dispersing (e.g., dispersing by adding water under high-speed stirring at 800-1000 r/min) and adding the component f for chain expansion reaction, and after the reaction is completed, removing the solvent (acetone) to obtain the polyurethane-polyurea aqueous dispersion. In a case where the component c contains the ionic group, the specific preparation steps include: reacting the component a, the component b, and the optional component d in the exist of a solvent (e.g., acetone) at 60-90°C to obtain the prepolymer containing isocyanate as terminal group, diluting with a solvent (e.g., acetone) and then adding the component c and the component f for further reaction, and then dispersing by adding water; removing the solvent (acetone) to obtain the polyurethane-polyurea aqueous dispersion.

The components or other related contents involved in the preparation of the polyurethane-polyurea aqueous dispersion in the above preparation method can be known with reference to the description of the polyurethane-polyurea aqueous dispersion hereinbefore, which will not be repeated herein in details.

In some embodiments, the polyurethane-polyurea aqueous dispersion prepared by the present application has a solid content of 35wt% or more, for example, 35-40wt%, a preferred pH value of 6 to 10, and an average particle size of, for example, 80-300 nm.

The present application also provides a soft elastic product, which is produced on the basis of the polyurethane-polyurea aqueous dispersion as described above; preferably, the soft elastic product may be a variety of products having soft and elastic properties, including but not limited to a glove and/or a condom.

The present application also provides a preparation method for a soft elastic product, including a step of subjecting a feeding liquid containing the polyurethane-polyurea aqueous dispersion of the present application as described above to a mold required to prepare a soft elastic product; preferably, the soft elastic product includes a glove and/or a condom. The specific steps for the preparation method for the soft elastic product can be carried out with reference to the corresponding existing conventional processes, but it is crucial that the polyurethane-polyurea aqueous dispersion provided by the present application is used in the feeding liquid. Where the preparation method for the soft elastic product is not specifically described, the skilled in the field can refer to the conventional process of preparing the corresponding soft elastic product in the field. Taking the preparation of a glove as an example, an exemplary preparation steps for reference includes: preparing the polyurethane-polyurea aqueous dispersion provided by the present application and other auxiliary materials into a feeding liquid, where the other auxiliary materials are, for example, water required for diluting the dispersion, and colorant, titanium dioxide, etc. added according to the needs for the product; immersing a glove mold in a solidifying solution (e.g., an aqueous solution containing 10wt% calcium chloride and 5wt% release agent (e.g., calcium stearate)) and removing out for drying, then immersing the glove mold in the above feeding liquid, removing out for drying (the operations of immersing in the feeding liquid and drying can be repeated based on the actual situation), immersing in a glove coating agent if needed, removing out for drying, and then demolding to obtain the finished glove.

The present application is illustrated exemplarily below through specific embodiments.

Some of the raw materials used in the examples and comparative examples are described below:
the polyols 1-3 and 7-8 are prepared according to the following synthesis method:
the required component I, diol, and catalyst are added to a reaction kettle by their respective formula amounts, the system is heated up to 140°C until the system is transparent, then the system is heated up to 210-220°C for reacting (the reaction is carried out for about 2-3 h) until the water output is close to the theoretical water output, then the system is heated up to 235-240°C and the vacuum device is turned on, the system is reacted under the vacuum pressure of -0.095 Mpa until the acid value of the system reaches 0.3 mgKOH/g or lower, and cooled down to 120°C, and the product is discharged and packaged to obtain the polyester polyol.

Polyol 1 (semi-crystalline polyester polyol): poly(hexanedioic acid-isophthalic acid-1,4-butanediol ester) (wherein hexanedioic acid and isophthalic acid are used by a molar ratio of 2.3: 1), having a functionality of 2, Mn = 2000, and being an opaque waxy solid at room temperature; in the preparation of the polyol 1, 1,4-butanediol and the component I have a molar ratio of 1.21: 1, and the catalyst is tetrabutyl titanate of 100 ppm.

Polyol 2 (semi-crystalline polyester polyol): poly(hexanedioic acid-isophthalic acid-1,6-hexanediol ester) (wherein hexanedioic acid and isophthalic acid are used by a molar ratio of 3.0: 1), having a functionality of 2, Mn = 2000, and being an opaque waxy solid at room temperature; in the preparation of the polyol 2, 1,6-hexanediol and the component I have a molar ratio of 1.27: 1, and the catalyst is tetrabutyl titanate of 100 ppm.

Polyol 3 (semi-crystalline polyester polyol): poly(hexanedioic acid-isophthalic acid-1,4-butanediol-neopentanediol ester) (wherein hexanedioic acid and isophthalic acid are used by a molar ratio of 4.5: 1), having a functionality of 2, Mn = 3000, and being an opaque waxy solid at room temperature; in the preparation of the polyol 3, a molar ratio of the total amount of 1,4-butanediol and neopentanediol to the component I is 1.17: 1, the catalyst is tetraisopropyl titanate of 150 ppm, and 1,4-butanediol and neopentanediol have a molar ratio of 7:3.

Polyol 4: polyhexanedioic acid-1,4-butanediol polyester-neopentanediol ester, having a functionality of 2, Mn = 3000, semi-crystalline, being an opaque waxy solid at room temperature; product number: WANTHANOL^{®}WHP-3045.

Polyol 5: poly(hexanedioic acid-neopentanediol ester), having a functionality of 2, Mn = 2000, semi-crystalline; product number: WANTHANOL^{®}WHP-205.

Polyol 6: poly(hexanedioic acid-neopentanediol-1,6-hexanediol ester), having a functionality of 2, Mn = 1500, Wanhua Chemical Group Co., Ltd, semi-crystalline, being an opaque waxy solid at room temperature; product number: WANTHANOL^{®}WHP-1556. Polyol 7: poly(hexanedioic acid-isophthalic acid-1,4-butanediol ester) (wherein hexanedioic acid and isophthalic acid have a molar ratio of 1.7: 1), having a functionality of 2, Mn = 2000, semi-crystalline, and being an opaque waxy solid at room temperature; in the preparation of the polyol 7, 1,4-butanediol and the component I have a molar ratio of 1.25: 1, and the catalyst is tetrabutyl titanate of 100 ppm. Polyol 8: poly(hexanedioic acid-isophthalic acid-1,4-butanediol ester) (wherein hexanedioic acid and isophthalic acid have a molar ratio of 0.8: 1), having a functionality of 2, Mn = 2000, semi-crystalline, and being an opaque waxy solid at room temperature; in the preparation of the polyol 8, 1,4-butanediol and the component I have a molar ratio of 1.235: 1, and the catalyst is tetrabutyl titanate of 130 ppm.

Isophorone diamine, hexamethylene diisocyanate (HDI), 4,4'-dicyclohexylmethane diisocyanate (HMDI), isophorone diisocyanate (IPDI), toluene diisocyanate (TDI), the aqueous solution of 50% sodium N-(2-aminoethyl)-2-aminoethanesulfonate, and neopentyl glycol are all from Wanhua Chemical Group Co., Ltd;
Polycarbonate: Mn = 2000, having a functionality of 2, Japan Polyurethane Co., Ltd;
Polytetrahydrofuran diol: Mn = 2000, having a functionality of 2, Mitsubishi Chemical Corporation, Japan;
Polycaprolactone diol: Mn = 2000, having a functionality of 2, Daicel Corporation, Japan;
Dimethylolpropionic acid (DMPA): Perstorp;
1,4-Butanediol, ethylenediamine, triethylamine: Sinopharm Chemical Reagent Co.,Ltd.

### Example 1

The 422.6 g of polyol 1, 78 g of polyol 3, 7.7 g of IPDI, 78 g of HDI, 7.16 g of DMPA, 4.0 g of 1,4-butanediol, and 110 g of acetone were added to a 1 L four-neck round-bottom flask equipped with nitrogen inlet and outlet, and the mixture was stirred at 80-90°C until the NCO reached 1.92wt%. The mixture was cooled to 40-45°C and diluted by adding 500 g of acetone, then neutralized by adding 4.8 g of triethylamine for about 5 min, and then dispersed by adding 900 g of water, and added with 5 g of ethylenediamine with stirring and subjected to a post-chain expansion reaction for 15 min. Subsequently, after separating the acetone by distillation, a solvent-free dispersion was obtained which had a solid content of 40wt%, an average particle size of 148 nm, and a pH of 8.3.

### Example 2

The 400 g of polyol 1, 48 g of IPDI, 60 g of HDI, 10 g of DMPA, 8.0 g of 1,4-butanediol, and 120 g of acetone were added to a 1 L four-neck round-bottom flask equipped with nitrogen inlet and outlet, and the mixture was stirred at 80-90°C until the NCO reached 2.69wt%. The mixture was cooled to 40-45°C and diluted by adding 500 g of acetone, then neutralized by adding 7.0 g of triethylamine for about 5 min, and then dispersed by adding 800 g of water, and added with 6.5 g of ethylenediamine with stirring and subjected to a post-chain expansion reaction for 15 min. Subsequently, after separating the acetone by distillation, a solvent-free dispersion was obtained which had a solid content of 40wt%, an average particle size of 140 nm, and a pH of 8.3.

### Example 3

The 400 g of polyol 1, 90 g of polytetrahydrofuran diol, 60 g of TDI, 30 g of HMDI, 10 g of DMPA, and 120 g of acetone were added to a 1 L four-neck round-bottom flask equipped with nitrogen inlet and outlet, and the mixture was stirred at 80-90°C until the NCO reached 1.63wt%. The mixture was cooled to 40-45°C and diluted by adding 500 g of acetone, then neutralized by adding 6.8 g of triethylamine for about 5 min, and then dispersed by adding 895 g of water, and added with 11.8 g of isophorone diamine with stirring and subjected to a post-chain expansion reaction for 15 min. Subsequently, after separating the acetone by distillation, a solvent-free dispersion was obtained which had a solid content of 40wt%, an average particle size of 170 nm, and a pH of 7.80.

### Example 4

The 300 g of polyol 2, 90 g of polyol 6, 55 g of TDI, 30 g of HMDI, 12 g of DMPA, and 90 g of acetone were added to a 1 L four-neck round-bottom flask equipped with nitrogen inlet and outlet, and the mixture was stirred at 80-90°C until the NCO reached 2.3wt%. The mixture was cooled to 40-45°C and diluted by adding 500 g of acetone, then neutralized by adding 8.1 g of triethylamine for about 5 min, and then dispersed by adding 895 g of water, and added with 11 g of isophorone diamine with stirring and subjected to a post-chain expansion reaction for 15 min. Subsequently, after separating the acetone by distillation, a solvent-free dispersion was obtained which had a solid content of 40wt%, an average particle size of 170 nm, and a pH of 8.0.

### Example 5

The 350 g of polyol 2, 90 g of polycarbonate, 55 g of HDI, 30 g of HMDI, 8 g of DMPA, 3 g of neopentyl glycol, and 90 g of acetone were added to a 1 L four-neck round-bottom flask equipped with nitrogen inlet and outlet, and the mixture was stirred at 80-90°C until the NCO reached 1.75wt%. The mixture was cooled to 40-45°C and diluted by adding 500 g of acetone, then neutralized by adding 5.43 g of triethylamine for about 5 min, and then dispersed by adding 825 g of water, and added with 11 g of isophorone diamine with stirring and subjected to a post-chain expansion reaction for 15 min. Subsequently, after separating the acetone by distillation, a solvent-free dispersion was obtained which had a solid content of 40wt%, an average particle size of 170 nm, and a pH of 7.90.

### Example 6

The 237.5 g of polyol 1, 328 g of polyol 4, 7.77 g of IPDI, 76 g of HDI, 8 g of DMPA, 4.0 g of 1,4-butanediol, and 110 g of acetone were added to a 1 L four-neck round-bottom flask equipped with nitrogen inlet and outlet, and the mixture was stirred at 80-90°C until the NCO reached 1.67wt%. The mixture was cooled to 40-45°C and diluted by adding 500 g of acetone, then neutralized by adding 5.4 g of triethylamine for about 5 min, and then dispersed by adding 1000 g of water, and added with 4.6 g of ethylenediamine with stirring and subjected to a post-chain expansion reaction for 15 min. Subsequently, after separating the acetone by distillation, a solvent-free dispersion was obtained which had a solid content of 40wt%, an average particle size of 138 nm, and a pH of 8.4.

### Example 7

The 240 g of polyol 2, 135 g of polyol 6, 60 g of TDI, 29 g of HMDI, 12 g of DMPA, 90 g of acetone were added to a 1 L four-neck round-bottom flask equipped with nitrogen inlet and outlet, and the mixture was stirred at 80-90°C until the NCO reached 2.28wt%. The mixture was cooled to 40-45°C and diluted by adding 500 g of acetone, then neutralized by adding 8.1 g of triethylamine for about 5 min, and then dispersed by adding 895 g of water, and added with 13 g of isophorone diamine with stirring and subjected to a post-chain expansion reaction for 15 min. Subsequently, after separating the acetone by distillation, a solvent-free dispersion was obtained which had a solid content of 40wt%, an average particle size of 188 nm, and a pH of 8.0.

### Example 8

The 220 g of polyol 2, 220 g of polycarbonate, 55 g of HDI, 30 g of HMDI, 8 g of DMPA, 3 g of neopentyl glycol, and 90 g of acetone were added to a 1 L four-neck round-bottom flask equipped with nitrogen inlet and outlet, and the mixture was stirred at 80-90°C until the NCO reached 1.75wt%. The mixture was cooled to 40-45°C and diluted by adding 500 g of acetone, then neutralized by adding 5.43 g of triethylamine for about 5 min, and then dispersed by adding 825 g of water, and added with 11 g of isophorone diamine with stirring and subjected to a post-chain expansion reaction for 15 min. Subsequently, after separating the acetone by distillation, a solvent-free dispersion was obtained which had a solid content of 40wt%, an average particle size of 163 nm, and a pH of 8.0.

### Example 9

The 450 g of polyol 1, 52.1 g of HDI, 30.2 g of IPDI, 2.6 g of neopentyl glycol, and 67 g of acetone were added to a 1 L four-neck round-bottom flask equipped with nitrogen inlet and outlet, and the mixture was stirred at 80-90°C until the NCO reached 2.8%. The mixture was cooled to 40-50°C and diluted by adding 740 g of acetone, added with 19 g of aqueous solution of 50wt% sodium N-(2-aminoethyl)-2-aminoethanesulfonate diluted by water of 5 times volume and 13 g of isophorone diamine with stirring and subjected to a reaction for 15 min, and dispersed by adding 800 g of deionized water with stirring. Subsequently, after separating the acetone by distillation, a solvent-free dispersion was obtained which had a solid content of 40wt%, an average particle size of 218 nm, and a pH of 7.5.

### Example 10

The 350 g of polyol 1, 250 g of polytetrahydrofuran diol, 69 g of HDI, 33 g of HMDI, and 87 g of acetone were added to a 1 L four-neck round-bottom flask equipped with nitrogen inlet and outlet, and the mixture was stirred at 80-90°C until the NCO reached 2.5wt%. The mixture was cooled to 40-50°C and diluted by adding 740 g of acetone, added with 22 g of aqueous solution of 50wt% sodium N-(2-aminoethyl)-2-aminoethanesulfonate diluted by water of 5 times volume and 10 g of isophorone diamine with quick stirring and subjected to a reaction for 15 min, and dispersed by adding 900 g of deionized water with stirring. Subsequently, after separating the acetone by distillation, a solvent-free dispersion was obtained which had a solid content of 40wt%, an average particle size of 258 nm, and a pH of 7.2.

### Comparative Example 1

The polyol 2 in Example 5 was changed to polycarbonate; the other remained unchanged. The resulting dispersion had a solid content of 40wt%, an average particle size of 161 nm, and a pH of 7.90.

### Comparative Example 2

The 150 g of polyol 1, 580 g of polyol 4, 48 g of IPDI, 60 g of HDI, 10 g of DMPA, 6.0 g of 1,4-butanediol, and 120 g of acetone were added to a 1 L four-neck round-bottom flask equipped with nitrogen inlet and outlet, and the mixture was stirred at 80-90°C until the NCO reached 1.39wt%. The mixture was cooled to 40-45°C and diluted by adding 500 g of acetone, then neutralized by adding 6.7 g of triethylamine for about 5 min, and then dispersed by adding 1290 g of water, and added with 4.8 g of ethanediamine with stirring and subjected to a post-chain expansion reaction for 15 min. Subsequently, after separating the acetone by distillation, a solvent-free dispersion was obtained which had a solid content of 40wt%, an average particle size of 178 nm, and a pH of 8.3.

### Comparative Example 3

The polyol 1 in Example 2 was changed to polycaprolactone diol; the other remained unchanged. The resulting dispersion had a solid content of 40wt%, an average particle size of 140 nm, and a pH of 8.4.

### Comparative Example 4

The polyol 1 in Example 2 was changed to polytetrahydrofuran diol; the other remained unchanged. The resulting dispersion had a solid content of 40wt%, an average particle size of 130 nm, and a pH of 8.1.

### Comparative Example 5

The polyol 1 in Example 2 was changed to the polyol 7; the other remained unchanged. The resulting dispersion had a solid content of 40wt%, an average particle size of 144 nm, and a pH of 8.2.

### Comparative Example 6

The polyol 1 in Example 2 was changed to the polyol 8; the other remained unchanged. The resulting dispersion had a solid content of 40wt%, an average particle size of 151 nm, and a pH of 8.4.

**Preparation of glove matrix resin (material liquid):** the dispersion prepared above was diluted with deionized water to reach a solid content of 15wt%, then added with 0.5wt% of colorant and 5wt% of titanium dioxide, and stirred at a low speed (within 200-300 r/min) for half an hour and set aside for later use.

**Preparation of solidifying solution:** an aqueous solution containing 10wt% CaCl₂ and 5wt% release agent (calcium stearate) was prepared with deionized water, stirred continuously for 1 h, and set aside for later use.

### Preparation of glove:

1) A cleaned glove mold was dried by an oven at 100°C.
2) The glove mold was taken out and cooled down to 60°C, immersed into the solidifying solution for about 8 s and taken out, and placed in an oven at 120-140°C and dried.
3) The dried glove mold was taken out and cooled down to 60°C, immersed into the matrix resin for about 8 s,
   taken out the mold and placed in an oven at 130°C and dried for about 4 min, then taken out and immersed into the matrix resin again for about 8 s, and then placed in an oven and baked for about 20 min.
4) The baked glove mold was taken out, then immersed into a glove coating agent, drawn out and dried in the air, and after mold releasing, the finished glove was obtained.

### Performance test:

Tensile strength and elongation test: The palm part of the glove was cut into a dumbbell shape with a width of 6 mm and a length of 115 mm, and tested by a tensile tester for the tensile strength and also elongation at the same time.

Water resistance test: The glove was filled with 800 g of water, inverted, and inspected for its deformation and appearance.

Oxides resistance: The glove was filled with 30wt% hydrogen peroxide, held for 10 min, and inspected for leakage or seepage conditions.

Polar solvent resistance: The glove was filled with a 75wt% ethanol solution, held for 10 min, and inspected for leakage or seepage conditions.

Resilience and wearing tactility: using the "commercially available latex gloves" listed in Table 1 as a reference, evaluate the resilience and wearing tactility performance of the gloves obtained in examples and comparative examples during the wearing.

The performance test results are shown in Table 1.

**Table 1**

| | Tensile strengt h /MPa | elongatio n/% | Resilience | Wearing tactility | Water resistance | Oxides resistance | Polar solvent resistance |
|---|---|---|---|---|---|---|---|
| Example 1 | 45 | 620 | Excellent | Soft and elastic | No obvious change | No leakage nor seepage | No leakage nor seepage |
| Example 2 | 52 | 650 | Excellent | Soft and elastic | No obvious change | No leakage nor seepage | No leakage nor seepage |
| Example 3 | 48 | 600 | Excellent | Soft and elastic | No obvious change | No leakage nor seepage | No leakage nor seepage |
| Example 4 | 55 | 630 | Excellent | Soft and elastic | No obvious change | No leakage nor seepage | No leakage nor seepage |
| Example 5 | 49 | 678 | Excellent | Soft and elastic | No obvious change | No leakage nor seepage | No leakage nor seepage |
| Example 6 | 45.8 | 620 | Good | Poor performance e in being soft and elastic | No obvious change | No leakage nor seepage | No leakage nor seepage |
| Example 7 | 47 | 612 | Good | Poor performance e in being soft and elastic | No obvious change | No leakage nor seepage | No leakage nor seepage |
| Example 8 | 46 | 600 | Good | Poor performance e in being soft and elastic | No obvious change | No leakage nor seepage | No leakage nor seepage |
| Example 9 | 49 | 650 | Excellent | Soft and elastic | No obvious change | No leakage nor seepage | No leakage nor seepage |
| Example 10 | 45 | 660 | Good | Soft and elastic | No obvious change | No leakage nor seepage | No leakage nor seepage |
| Compara tive Example 1 | 45 | 370 | Poor | Relatively hard; plastic-like texture | No obvious change | No leakage nor seepage | No leakage nor seepage |
| Compara tive Example 2 | 47 | 400 | Poor | Relatively hard; plastic-like texture | No obvious change | Slight leakage and seepage | Slight leakage and seepage |
| Compara tive Example 3 | 40 | 377 | Poor | Relatively hard; plastic-like texture | No obvious change | Slight leakage and seepage | Slight leakage and seepage |
| Compara tive Example 4 | 30 | 550 | Good | Soft and elastic | Showing white color and deformatio n | Leakage and seepage | Leakage and seepage |
| Compara tive Example 5 | 43 | 400 | Poor | Relatively hard; plastic-like texture | No obvious change | Slight leakage and seepage | Slight leakage and seepage |
| Compara tive Example 6 | 44 | 410 | Poor | Relatively hard; plastic-like texture | No obvious change | Slight leakage and seepage | Slight leakage and seepage |
| Commer cially available butyronit rile gloves | 44 | 360 | Poor | Relatively hard; plastic-like texture | No obvious change | Slight leakage and seepage | Slight leakage and seepage |
| Commer cially available latex gloves | 24 | 620 | Excellent | Soft and elastic | Slight deformatio n | Slight leakage and seepage | Slight leakage and seepage |

As can be seen from the table, the gloves prepared on the basis of the polyurethane-polyurea aqueous dispersion of the present application are significantly superior to the gloves in comparative examples or the commercially available butyronitrile gloves in terms of the overall performance of tensile strength, elongation, resilience, wearing tactility, water resistance, etc., and significantly superior to the commercially available latex gloves in terms of tensile strength and water resistance.

It is understandable that the above embodiments are merely examples used for clear description but do not imply that the present application is limited thereto. A person of ordinary skill in the art can make variations or changes in various forms on the basis of the above description. It is neither necessary nor possible to exhaustedly list all the embodiments herein. The obvious variations or changes derived therefrom remain within the protection scope of the present application.

## Claims

1. A polyurethane-polyurea aqueous dispersion, comprising a unit derived from a component a; the component a comprises at least one polyol having a functionality of 2 or more, and the component a at least comprises 40wt% or more of a semi-crystalline polyester polyol obtained by reacting a diol with a component I, wherein the component I comprises an aliphatic dicarboxylic acid and a component II by a molar ratio of 2.1-10: 1, and the component II is an aromatic dicarboxylic acid and/or an aromatic dicarboxylic anhydride.

2. The polyurethane-polyurea aqueous dispersion according to claim 1, wherein the diol used to prepare the semi-crystalline polyester polyol at least comprises one or more of 1,4-butanediol and 1,6-hexanediol, and optionally comprises an additional diol having a relative molecular mass of less than 150; the additional diol comprises one or more of ethylene glycol, 1,3-butanediol, neopentyl glycol, 1,5-pentanediol, propylene glycol, diethylene glycol, and dipropylene glycol; the additional diol accounts for less than 50wt% of the diol used to prepare the semi-crystalline polyester polyol;
and/or, the component II used to prepare the semi-crystalline polyester polyol is selected from one or more of benzenedicarboxylic acid and phthalic anhydride; preferably, the benzenedicarboxylic acid is selected from isophthalic acid and/or phthalic acid; more preferably, the component II is isophthalic acid;
and/or, the aliphatic dicarboxylic acid used to prepare the semi-crystalline polyester polyol is hexanedioic acid.

3. The polyurethane-polyurea aqueous dispersion according to claim 1 or 2, wherein the semi-crystalline polyester polyol is an opaque waxy solid at room temperature.

4. The polyurethane-polyurea aqueous dispersion according to any one of claims 1-4, wherein the polyurethane-polyurea aqueous dispersion is prepared by reacting a raw material comprising the following components:
component a: comprising at least one polyol having a functionality of 2 or more; and the semi-crystalline polyester polyol accounts for 40wt% or more of the component a, and preferably the semi-crystalline polyester polyol accounts for 70-100wt% of the component a;
component b: a polyisocyanate;
component c: a hydrophilic compound; the hydrophilic compound contains 2-3 groups having reactivity with NCO, and a hydrophilic group of the hydrophilic compound comprises one or more of an ionic group and a latent ionic group;
optional component d: an alcohol chain extender different from the component a; and
component f: a polyamine having a number average molecular mass of 500 g/mol or less.

5. The polyurethane-polyurea aqueous dispersion according to claim 4, wherein
in a case where the hydrophilic group of the component c does not comprise the ionic group, the raw material further comprises component e, and the component e is a compound capable of ionizing the component c;
preferably, in a case where the raw material comprises the component e, relative to the total weight of the components a, b, c, d, e, and f, proportions of each component used to prepare the polyurethane-polyurea aqueous dispersion are as follows: 56-85wt% of the component a, 10-30wt% of the component b, 0.8-4wt% of the component c, 0-3wt% of the component d, 0.5-4wt% of the component f, and 0.6-4wt% of the component e;
preferably, in a case where the raw material does not comprise the component e, relative to the total weight of the components a, b, c, d, and f, proportions of each component used to prepare the polyurethane-polyurea aqueous dispersion are as follows: 56-85wt% of the component a, 10-30wt% of the component b, 0.8-4wt% of the component c, 0-3wt% of the component d, and 0.5-4wt% of the component f.

6. The polyurethane-polyurea aqueous dispersion according to claim 5, wherein in the component a, the polyol has a number average molecular mass of 500-15000 g/mol, preferably the polyol is a polyol having a number average molecular mass of 800-10000 g/mol and a functionality of 2 to 4, and more preferably the polyol is a polyol having a number average molecular mass of 1000-5000 g/mol and a functionality of 2 to 3;
and/or, in the component b, the polyisocyanate is selected from one or more of an aromatic polyisocyanate, an aliphatic polyisocyanate, and an alicyclic polyisocyanate, and preferably the polyisocyanate has at least two isocyanate groups;
and/or, in the component c, the groups having reactivity with NCO contained in the hydrophilic compound are hydroxyl and/or amino;
and/or, in the component c, the ionic group is preferably a carboxylate ion and/or a sulfonate ion, and the latent ionic group is preferably carboxyl;
and/or, in the component d, the alcohol chain extender different from the component a is a small-molecule alcohol compound having a relative molecular mass of less than 150;
and/or, the component e is triethylamine;
and/or, the component f is a polyamine having a relative molecular mass of 60-500, and preferably one or more of ethylenediamine, 1,2-diaminopropane, 1,4-diaminobutane, 1,6-hexanediamine, 2-methylpentane-1,5-diamine, isophorone diamine, 4,4-diaminodicyclohexylmethane, piperazine, and diethylenetriamine.

7. The polyurethane-polyurea aqueous dispersion according to claim 6, wherein the hydrophilic compound used as the component c is selected from one or more of dimethylolpropionic acid, dimethylolbutanoic acid, dihydroxysuccinic acid, N-(2-aminoethyl)-2-aminoethanesulfonic acid, N-(3-aminopropyl)-2-aminoethanesulfonic acid, and salts thereof, the salts comprise one or more of their respective alkaline metal salts, alkaline earth metal salts, or ammonium salts, and preferably the component c is dimethylolpropionic acid;
and/or, the alcohol chain extender used as the component d is selected from one or more of ethylene glycol, 1,2-propanediol, 1,3-propanediol, 1,4-butanediol, 1,3-butanediol, 2,3-butanediol, 1,5-pentanediol, 1,6-hexanediol, neopentyl glycol, 1,4-dihydroxycyclohexane, 1,4-dimethylolcyclohexane, 1,8-octanediol, 1,10-decanediol, 1,12-dodecanediol, neopentyl glycol, 1,4-cyclohexanediol, 1,4-cyclohexanedimethanol, and 2-ethyl-1,3-hexanediol, and preferably one or more of 1,2-propanediol, 1,3-propanediol, 1,4-butanediol, 1,3-butanediol, neopentyl glycol, 1,4-cyclohexanedimethanol, and 1,6-hexanediol.

8. The polyurethane-polyurea aqueous dispersion according to claim 6, wherein the component a optionally comprises an additional polyol different from the semi-crystalline polyester polyol, the additional polyol is selected from one or more of a polyester polyol, a polycaprolactone polyol, a polycarbonate polyol, and a polyether polyol, and the additional polyol accounts for 0-60wt% of the component a.

9. A preparation method for the polyurethane-polyurea aqueous dispersion according to any one of claims 1-8, wherein the polyurethane-polyurea aqueous dispersion is prepared by reacting a raw material comprising the component a;
preferably, the raw material further comprises component b, component c, optional component d, and component f;
wherein the component b is a polyisocyanate; the component c is a hydrophilic compound, and the hydrophilic compound contains 2-3 groups having reactivity with NCO; the component d is an alcohol chain extender different from the component a; the component f is a polyamine having a number average molecular mass of 500 g/mol or less; in a case where the component c does not comprise an ionic group, the raw material further comprises component e, and the component e is a compound capable of ionizing the component c;
preferably, the preparation method comprises a step of obtaining a prepolymer containing isocyanate as terminal group by reacting at 60-90°C.

10. A soft elastic product, which is produced from the polyurethane-polyurea aqueous dispersion according to any one of claims 1-8; preferably the soft elastic product comprises a glove and/or a condom.

11. A method for preparing a soft elastic product, comprising a step of subjecting a feeding liquid containing a polyurethane-polyurea aqueous dispersion to a mold required to prepare a soft elastic product; preferably, the soft elastic product comprises a glove and/or a condom; the polyurethane-polyurea aqueous dispersion employs the polyurethane-polyurea aqueous dispersion according to any one of claims 1-8.
